Europäisches Patentamt

European Patent Office

Office européen des brevets

(11) Publication number : 0 510 619 A1

# EUROPEAN PATENT APPLICATION

(21) Application number : 92106892.0

(22) Date of filing : 22.04.92

(51) Int. Cl.⁵ : A61F 13/15

(30) Priority : 22.04.91 US 689389

(43) Date of publication of application :
28.10.92 Bulletin 92/44

(84) Designated Contracting States :
BE DE ES FR GB IT NL SE

(71) Applicant : KIMBERLY-CLARK
CORPORATION
401 North Lake Street
Neenah, Wisconsin 54956-0349 (US)

(72) Inventor : Majors, Stephanie Ronilo
85 South Shore Drive
Newnan, Georgia (US)
Inventor : Owen, Geoffrey Robert
205 Spring Ridge Trace
Roswell, Georgia 30076 (US)
Inventor : Weber, Mary Garvie
125 Greenmont Downs Trace
Alpharetta, Georgia 30201 (US)

(74) Representative : Diehl, Hermann Dr.
Diehl & Glaeser, Hiltl & Partner
Flüggenstrasse 13
W-8000 München 19 (DE)

(54) Article for absorbing bodily fluids and controlling malodor created by same.

(57) An article (18) for absorbing bodily fluids and controlling malodor created by the presence of the bodily fluid. The article (18) includes a liquid permeable topsheet (20) and a liquid impermeable backsheet (24) forming an envelope or cavity with the topsheet. A liquid absorbent material (26) is disposed between the topsheet (20) and the backsheet (24). At least one bacterial growth inhibitor (30) is generally disposed between the topsheet (20) and the liquid absorbent material (26). An odor control complex is disposed between the topsheet (20) and the backsheet (24) and can include a sheet form odor control member (44). The odor control agents forming the odor control complex can be protected against moisture by various artifices, including hydrophobically coating the odor control agent, shielding the odor control agent with a liquid impermeable sheet (52), and disposing the odor control agent where it is less likely to be contacted by moisture. The selection of combination of odor control agents and their relative disposition within the article are designed to enhance their effectiveness in removing malodor from the article.

FIG. 3

EP 0 510 619 A1

The present invention relates to an article for absorbing bodily fluids and controlling madodor created by same.

Absorbent pads designed to be worn by humans to absorb bodily fluids such as urine, menstrual fluid, perspiration, etc., include such articles as disposable diapers, sanitary napkins, tampons, panty shields, and incontinence pads. In use, the absorbed bodily fluids become the source of malodors. In the case of sanitary napkins, these malodors result from the interactions among menstrual fluid, volatiles, and bacteria and their byproducts. Menstrual fluid contains acidic, basic, and pH neutral compounds, which can form malodors over a short time span. With some compounds, the odors can form as soon as the fluid exits the body. Consequently, a number of products have been developed for absorbing menstrual fluid and controlling certain of its quick forming malodors.

In addition, menstrual fluid which contains microorganisms can generate malodorous volatile byproducts over time frames of four hours or longer. Available products fail to sufficiently combat the entire spectrum of odor causing compounds and thus fail to control both quick forming odors as well as odors which develop over longer time periods associated with a significant presence of microorganisms.

Various compounds, chemicals, mixtures, and like materials (i.e., absorbents such as activated carbon, clay, zeolites and molecular sieves), are useful in controlling malodors. Certain types of these materials are more effective against specific types of malodorous compounds. For example, a basic buffered composition is more effective than an acidic or pH neutral composition in controlling acidic odors present in menstrual fluid. Some products include more than one agent to neutralize the various pH elements (acidic, basic, or neutral) of the malodorous compounds.

Examples of specific absorbent products providing odor control include a sanitary napkin disclosed in U.S. Patent No. 3,340,875 to Dudley et al. This product's odor absorbent media consist of an activated clay, or the like, in admixture with a cation exchange type solid material. The deodorizing media is a mixture of a neutral adsorbent material selected from the group consisting of activated carbon, activated alumina, activated silica, diatomaceous earth, infusorial clay and attapulgite clay and, in its preferred form, a polycarboxylic type ion-exchange resin. The resins are said to possess a special affinity for ammonia and the amines present in bacterial decomposition products and will absorb such gaseous compounds which may be generated within the napkin. The preferred resins are the AMBERLITES® from Rohm and Haas Company. The preferred location for the odor absorbent media is upon the interior surface of the outermost layer of crepe wadding in the napkin which will be placed toward the body. In particular, the top layer of wadding has dispersed over its surface facing the interior of the napkin, deodorizing media which may include a suitable bonding agent, such as polyvinyl alcohol.

U.S. Patent No. 4,661,101 to Sustmann discloses a catamenial device including a microbistatic or microbicidal and deodorizing fibrous core and an outer covering of an untreated, pH-regulating, or buffered fibrous, cellulose material. A major objective of the invention is to prevent the microbistatic agent from contacting the vaginal mucous. Thus, the agent is firmly anchored to the core, which includes a fibrous cellulose material modified by anionic moieties and finished with microbistatically active cations attached to those moieties. Suitable cations include heavy metal ions, ions of transition metals, quaternary ammonium ions or any mixture thereof. The core material can be maintained at a pH range of 4 to 5. The outer layer of this device may be a pH-regulating fibrous cellulosic material modified by carboxymethyl groups for maintaining the fiber pH value at 3 to 4. The outer layer may also be a buffer impregnated material containing citric acid, sodium hydroxide, and other ingredients having an optimum pH buffering range of about 4 to 6.

U.S. Patent 4,059,114 to Richards discloses a disposable shield utilizing an absorptive portion having an antimicrobial agent alone or in conjunction with a malodor counteractant incorporated therein. As the antimicrobial agent, a 1.25% isopropyl alcohol solution of "Ozone Bouquet" 96025 from Monsanto Co. was applied to the inner surface of the absorptive layer.

Canadian Patent No. 474,818 discloses a sanitary napkin which incorporates a quaternary ammonium salt into the cellulosic pad in the portion of the pad adjacent the surface which is initially contacted by the liquid body excretion. When the liquid body excretion contacts the quaternary ammonium compound, there is some reaction with the odor producing matter in the excretion which mitigates odors and which effectively prevents the subsequent development of odors.

U.S. Patent No. 4,289,513 to Brownhill et al discloses activated sorption paper capable of being incorporated into a sanitary napkin. The paper contains sorption particles which include activated carbon, activated silica gel, polymeric absorbance materials, molecular sieve ion exchange resins, and other carbonaceous materials, whereby the particles are substantially uniformly dispersed throughout all dimensions of the fibrous paper base material. A starch or starch-like binder is incorporated with the particles to form the paper. While the location of the paper insert within the sanitary napkin is optional, a placement intermediate the width of the absorbent pad is preferred.

U.S. Patent No. 3,794,034 to Jones discloses a body waste fluid absorbent pad, such as a sanitary napkin, whereby a substantial portion of the pad has a water soluble, weakly acidic, buffered solid composition having an aqueous pH range of 3.5 to 6.0 in the dry reservoir portion of the pad. The controlled pH range of the buffered pad inhibits the rapid formation of ammonia and volatile amines from the urea, uric acid, amino acids and peptones in the body waste fluids, by bacterial and enzyme action. The buffered pH acidic reservoir has a two-fold function. First, it inhibits bacterial growth in the proteolytic degradation of the excretory products. Secondly, the ammonia and amines,'which are initially excreted in the waste fluids, are immediately neutralized by the buffer composition, forming nonvolatile salts. Preferably, the buffer impregnated tissue sheets utilized in the sanitary napkin are disposed adjacent the surface of the napkin which first receives the menstrual fluid.

U.S. Patent No. 3,804,094 to Manoussos et al discloses a sanitary napkin including a body of an absorbent material having a fluid permeable outer portion containing periodic acid in amounts effective to deodorize fluid menstrual discharge over a prolonged period, even when the napkin has been removed for up to five hours. The entire absorbent material need not be impregnated or saturated with periodic acid. Only the fluid permeable outer portion which directly contacts the body need contain the periodic acid.

U.S. Patent No. 4,525,410 to Hagiwara et al discloses a fiber article packed with natural or synthetic zeolitic particles having at least one metal ion with a bactericidal property. Suitable applications of the fabric article include mats, beds, bedclothes, pillows, filters insoles of shoes, and general sanitary goods. The $SiO_2/Al_2O_3$ mole ratio of the zeolite should be at most 14, preferably 11. Thus, zeolites commonly known as molecular sieves are unsuitable.

U.S. Patent No. 4,372,309 to Fowler discloses moisture absorbent pads having incorporated therein any one of a number of known bacterial static agents for preventing biological degradation of urine and the formation of ammonia and amines. The absorbent may also include a substance (such as boric acid or citric acid) for absorbing volatile nitrogenous compounds.

U.S. Patent No. 4,381,784 to Aberson et al discloses an absorbent device, such as a sanitary napkin combined with a blood gelling agent, designed to absorb blood or blood-like fluids. The blood gelling agents are generally of two types: dicarboxylic anhydrides and polycations, and the agents work best at a pH range of 6.2 to 7.2. The blood gelling agent is disposed in conjunction with conventional fluid direction means such as embossed lines, uniformly with the absorbent component, in a uniform layer near the bottom of the napkin, or in a single intermediately positioned layer with or without suitable carriers.

U.S. Patent No. 3,856,014 to Yamauchi discloses a sanitary napkin for deodorizing malodorous liquid. The deodorant combats basic malodorous constituents, acidic malodorous constituents, and neutral malodorous constituents. The napkin contains at least one layer of staple fibers having adhered thereto a porous resin powder. The resinous powder is preferably in the form of an acid salt formed by neutralizing amino groups in the resin with acids, whereby the resinous powder has a water content of 30 to 50 per cent. The components of the napkin are arranged whereby two layers of staple fibers containing the deodorant are separated by at least one layer of water absorbing paper and surrounded on one side with at least one layer of water-repellent paper and on the other side with at least one layer of water absorbing paper. The entire napkin is housed within a non-woven fabric wrapper. Also, a sheet of staple fibers containing activated carbon powder may be used for either of the two sheets of staple fibers, and this deodorant powder may be disposed in the water absorbing paper between the two sheets of staple fibers.

U.S. Patent No. 2,690,415 to Shuler discloses a flexible odor-adsorbent sheet, which consists of granulated odor-adsorbent material adhered to an open gauze web with a water emulsion asphaltic type adhesive. The odor adsorbent material is preferably granulated activated carbon. The disposition of the adhesive upon the gauze web does not substantially impair the over-all porosity or fluid pervious nature of the gauze and permits the free flow of fluid through the gauze containing the particles.

U.S. Patent No. 4,583,980 to Schneider et al discloses a sanitary hygiene product having an absorbent layer of suitable fibrous material penetrated as uniformly as possible by the esters of citric acid and/or acetylcitric acid for preventing the local decomposition of the secretions taken up in the absorbent layer.

U.S. Patent No. 3,920,020 to Kraskin is directed to methods and means for controlling undesirable problems such as menstrual malodor, caused by formation of undesirable products on body surfaces and environs as a result of microbial action on lipoidal materials in body secretions. The malodors may be eliminated by applying to the situs of formation of the undesirable products, an inhibitory amount of an amino acid compound. The amino acid compounds are available as free acids, acid salts, and salts preferably in a situs of operation with a pH near neutrality, about 6.2 to 8.5. Although the amino acid compound may be distributed uniformly throughout a catamenial dressing, it is preferred to place it in that portion which first contacts the body discharge.

U.S. Patent No. 4,055,184 to Karami discloses an absorbent pad containing a solid finely-divided mixture of a hydrolyzed starch-polyacrylonitrile graft copolymer in acidic form and a water soluble basic material such as sodium bicarbonate disposed throughout a water absorbent mass such as cellulosic fibers. In another embodiment, the mixture is sprinkled between layers of absorbent fibrous material or on the faces thereof. The mixture can be concentrated at the upper face of the pad or at the bottom face of the body of the pad.

U.S. Patent No. 795,562 to Tatti discloses dress shields or insoles containing a compound for absorbing, neutralizing, and deodorizing sweat. The compound consists of a mixture of wood-charcoal, calcined magnesium or magnesium oxide, slaked lime or calcium hydrate, and zinc oxide.

U.S. Patent No. 4,715,857 to Juhasz et al discloses an antibacterial wound dressing that absorbs bacteria, reduces bacterial growth (by limiting oxygen availability), and provides a bacterial barrier, thereby minimizing external and cross-contamination. An activated charcoal fabric is disposed between two "enveloping" permeable material layers.

International Publication No. WO 81/01643 corresponding to International Application No. PCT/US80/01662 discloses a diaper incorporating an inorganic aluminosilicate zeolite ammonium ion exchange material for removing ammonia (and other toxic or potentially toxic nitrogenous irritants) from waste matter in the diaper. Additional capacity for removal of ammonia or other toxic substances can be achieved by the incorporation of activated carbon, amorphous permutite type aluminosilicates, crystalline aluminosilicates, or clay minerals. In the case where sodium exchanged zeolites are used, the pH range of the urine in the wet diaper can be controlled between 6 and 8 by the use of buffers. The active ingredients are evenly dispersed within the fibrous interlayer of a conventional disposable diaper.

U.S. Patent No. 2,024,145 to Cline discloses a napkin incorporating a deodorant in liquid form in mixture with a substance acting as a binder, whereby the mixture is sprayed upon or otherwise incorporated into the material constituting the napkin. Additionally, the mixture can be applied to a strip of gauze material forming a middle layer of the napkin.

Union Carbide brochure "Abscents--A new Approach for Odor Control" by Anthony J. Gioffre, copyright 1988, discloses that synthetic zeolites, or molecular sieves, can contribute to odor control and specifically could be used in feminine care products including menstrual pads and tampons. A sampling of the classes of odor causing compounds that ABSCENTS can effectively control includes organic acids, aldehydes, ketones, mercaptans, ammonia, and indoles. Test results with triethylamine and isovaleric acid illustrate that ABSCENTS™ removes these odorous compounds to low concentrations below threshold detection values.

An article by Ward Worthy, entitled, "New Molecular Sieves Eliminate Odors," C&EN, May, 1988, discloses that Union Carbide is marketing synthetic molecular sieves under the ABSCENTS™ and SMELLRITE™ names for use in various articles including menstrual pads.

While the documents discussed above may disclose products which exhibit some of the characteristics of the present invention, none of them discloses or suggests the present invention (including the advantages thereof), which achieves the objectives as discussed below and one of them being to provide an improved article for absorbing bodily fluids and controlling malodor created by the presence of the bodily fluid in the article.

The invention therefore provides such an article as evident from one of the independent claims 1, 13, 14, 15, and 18. Further advantageous features, aspects and details of the invention are evident from the dependent claims, the description, examples, tables and the drawings. The claims are intended to be understood as a first non-limiting approach of defining the invention in general terms.

The invention provides a multicomponent odor control device and an absorbent device that controls odors and which, for example, can be a sanitary napkin containing more than- one component for controlling odors.

DEFINITION

As used herein the term "test volatile mixture" refers to a mixture consisting of: (1) 100 microliters di-n-propyl sulfide; (2) 100 microliters of furaldehyde; (3) 100 microliters of triethylamine; (4) 200 microliters of isovaleric acid and (5) 100 microliters of pyridine.

To achieve the objects and in accordance with the purpose of the present invention, as embodied and broadly described herein, an article is provided for absorbing bodily fluids and controlling odor created by the presence of the bodily fluid in the article. The article is meant to be worn by the user and typically covers a portion of the user's anatomy, and in this sense the article can be referred to as a garment. Such article incorporates a multiple component odor control system of odor control agents disposed to control acidic, neutral, and basic malodors, and to inhibit the growth of odor causing bacteria. A specific example of an article according to the present invention includes a liquid permeable topsheet or cover; a liquid impermeable backsheet or baffle which can be joined to the cover to form an envelope or cavity; a liquid absorbent material disposed in the cavity formed between the topsheet and the backsheet, the liquid absorbent material being positioned to absorb liquid passed through the topsheet; at least one bacterial growth inhibitor generally disposed between the topsheet and the liquid absorbent material, whereby liquid passed through the topsheet generally contacts the bacterial growth inhibitor prior to the liquid absorbent material; and an odor control complex disposed between the topsheet and the backsheet; the odor control complex includes odor control agents selected so that in combination with all of said bacterial growth inhibitors, malodors arising from the article become reduced to levels undetectable by the human olfactory sense for a period of time of at least about four hours.

The topsheet desirably includes a liquid permeable material that allows passage of bodily fluid therethrough. The topsheet can be apertured and desirably is provided in one or more of the forms including: woven webs, nonwoven webs, spunbond nonwoven webs, meltblown webs, spunbond-meltblown webs, and coforms of one or more of the foregoing. The materials used to form the foregoing webs can be of any type without regard to crystalline or noncrystalline characteristics, as long as they can be spun into filaments. Examples of suitable materials include polyolefins such as polypropylene.

The backsheet can be in the form of a sheet or film of polymeric material that is impervious to bodily fluid and so prevents such liquid from escaping and soiling undergarments or the like. The backsheet can be flexible and desirably is formed of a polyethylene film that is impervious to both liquid and vapor. However, another alternative embodiment for the backsheet is a vapor permeable, but body fluid impermeable, layer of a blown microfiber web such as a spunbonded polypropylene web. In a still further alternative embodiment, the backsheet could be formed of a fiber-reinforced layer of a rubbery film-forming polymer such as butadiene-styrene.

The liquid absorbent material includes one or more layers of fluffed cellulose pulp material conventionally used in sanitary pads, diapers and the like. Desirably, the liquid absorbent material of the present invention can include superabsorbents, either alone or combined with fluff. Other suitable liquid absorbent materials include cellulosic derivatives such as rayon and cotton, synthetic polymers, and synthetic polymer blends such as polyester, polypropylene, nylon, polyethylene, and the like.

The bacterial growth inhibitor is capable of inhibiting the growth of bacteria present in menstrual fluid and in the article itself while not disturbing the normal flora present on the skin's surface. The bacterial growth inhibitor includes one or more of the inhibitors taken from the group including, but not limited to: chlorhexidine, quaternary ammonium compounds, cupric salts, chelating agents, parabens, chitin, and pH buffered materials.

The odor control agents selected to form the odor control complex desirably include a combination of at least two agents taken from the group including: natural zeolites, synthetic zeolites (such as, for example, aluminosilicate molecular sieve powders), activated carbon, activated clay, activated alumina, silica gels, acidic buffered pulp material, basic buffered pulp material, neutral buffered pulp material, cellulose web materials, chitin, polymeric resin materials, ion exchange resins, polymeric resin adsorbents of insoluble cross linked polymers in bead form, elastomeric meltblown material, elastomeric polystyrene material, and peat moss.

In some embodiments of the present invention, the effectiveness of the odor control agents is enhanced by disposing the liquid absorbent material between the topsheet and the odor control agents in a shielding manner that reduces exposure of the agents to bodily fluid. Accordingly, the odor control agents which benefit most from the shielding disposition of the liquid absorbent material include natural zeolites, synthetic zeolites (such as, for example, aluminosilicate molecular sieve powders), activated carbon, and polymeric resin adsorbents of insoluble cross-linked polymers in bead form.

In other embodiments of the invention, the odor control agents are disposed within the absorbent material in a relative orientation to each other that enhances their odor control capabilities. For example, a pH buffered pulp is integrated with a fluff layer which forms the liquid absorbent material, and at least one molecular sieve is disposed generally towards the backsheet. Another example is similar to the previous example, except that the molecular sieve has been hydrophobically coated and may be disposed generally homogeneously throughout the absorbent material. Two other examples are similar to the two previous examples, except that in each case the liquid absorbent material may also contain chitin disposed therein generally towards the topsheet, thereby enabling the chitin to directly contact the bodily fluid and gelatinize such waste fluid. Yet two further examples are similar to the first two examples, except that, in each case, the chitin may also be hydrophobically coated and disposed generally homogeneously throughout the liquid absorbent material.

In other embodiments of this invention, a top fluff layer of basic buffered pulp and a bottom fluff layer of acidic buffered pulp are provided. A molecular sieve may be disposed between the top and bottom layers of buffered pulps. The molecular sieve may be hydrophobically coated to reduce the wetting thereof by bodily fluid. Additionally, chitin may be disposed in the top fluff layer and concentrated generally in the vicinity of the top fluff layer near the topsheet, thereby enabling the chitin to gelatinize any waste fluid directly contacted by the chitin. In an alternative embodiment, the chitin may also be hydrophobically coated and disposed generally homogeneously throughout the top fluff layer.

In still other embodiments, the invention further includes a vapor permeable, water repellent web disposed between the liquid absorbent material and the molecular sieve odor control agent to further protect the molecular sieve from wetting.

In still further embodiments including a topsheet, backsheet, liquid absorbent material, and a bacterial growth inhibitor, the invention further includes a sheet form odor control member, which can include a molecular sieve integrated with a carrier substrate. The substrate may be selected from the group of substrates including: spunbond substrates, spunbond-meltblown substrates, spunbond-meltblown-spunbond substrates, and cellulose webs. Furthermore, the molecular sieve is in particulate form and adhered to the substrate by a suitable binder such as polyvinyl alcohol or latex. In a further refinement of these embodiments, the article includes a vapor permeable, water repellent web, and the sheet form odor control member is directly adjacent to the water repellent web. This sandwiches a polymeric resin of insoluble cross linked polymers in bead form between the water repellent web and the sheet form odor control member.

Other embodiments of the present invention are in the form of a sanitary napkin for absorbing menstrual liquid and controlling odor created by the presence of the bodily fluid in the napkin. The napkin includes, in the following relative order: a liquid permeable topsheet in the form of a spunbond web; a bacterial growth inhibiting meltblown web carrying a solution of chlorhexidine digluconate; a liquid absorbent material positioned to absorb liquid passed through the topsheet and the bacterial growth inhibiting web, the absorbent material including: a buffered pulp fluff layer further including citric acid and its trisodium salt and having a pH of about 5.5, a second pulp fluff layer, and hydrophobically coated chitin disposed between the buffered pulp layer and the second pulp fluff layer; a vapor permeable water repellent web including meltblown fibers formed from a blend of an olefin and a styrene-ethylene-butylene-styrene block copolymer; a particulate polymeric resin including a resin of insoluble cross linked polymers in bead form; a cellulosic web having particles joined thereto by a polyvinyl alcohol binder, the particles being selected from the group of molecular sieves including natural zeolites, synthetic zeolites, such as, for example, aluminosilicate molecular sieve powders, activated clay, activated carbon, activated alumina, and silica gels; and a liquid impermeable backsheet; wherein the vapor permeable water repellent web is adapted to reduce contact of menstrual liquid with the molecular sieve carried on the cellulosic web.

The accompanying drawings, which are incorporated in and constitute a part of this specification, illustrate embodiments of the invention and, together with the description, serve to explain the principles of the invention.

Fig. 1 is a schematically presented, perspective view of one embodiment of the present invention with sections cut away for ease of viewing components of the embodiment that otherwise would be hidden from view.

Fig. 2 is another schematic representation of a perspective view of another embodiment of the present invention with sections cut away for ease of viewing components of the embodiment that otherwise would be hidden from view.

Fig. 3 is a schematically presented, perspective view of yet another embodiment of the present invention with sections cut away for ease of viewing components of the embodiment that otherwise would be hidden from view.

Fig. 4 is yet another schematic representation of a perspective view of still another embodiment of the invention with sections cut away for ease of viewing components of the embodiment that otherwise would be hidden from view.

Fig. 5 is another schematic perspective view of one other embodiment of the invention with sections cut away for ease of viewing components of the embodiment that otherwise would be hidden from view.

Fig. 6 is a schematic representation of a perspective view of yet another embodiment of the present invention with sections cut away for ease of viewing components of the embodiment that otherwise would be hidden from view.

Fig. 7 is a schematically presented, perspective view of an article made in accordance with the present invention with sections cut away for ease of viewing components of the article that otherwise would be hidden from view.

Reference now will be made in detail to the presently preferred embodiments of the present invention, one or more examples of which are illustrated in the accompanying drawings. Each example is provided as an explanation and not a limitation of the invention. In fact, it will be apparent to those skilled in the art that various modifications and variations can be made in the present invention without departing from the scope or spirit of the invention. For instance, features illustrated or described as part of one embodiment, can be used on another embodiment to yield a still further embodiment. Thus, it is intended that the present invention cover all modifications and variations of this invention, provided they come within the scope of the appended claims and their equivalents.

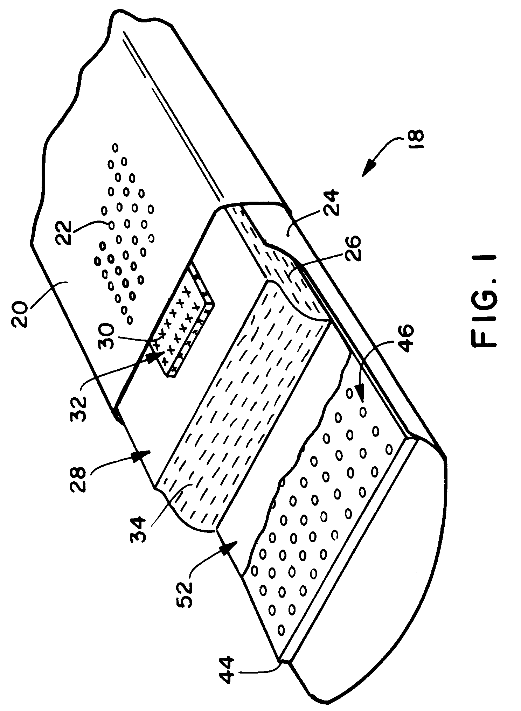

The present invention contemplates an article for absorbing bodily fluids and controlling odor created by the presence of the bodily fluid in the article. In embodiments of the invention as shown for example in Figs. 1-7, an article in accordance with the scope and spirit of the present invention is generally designated by the numeral 18. The article can be a feminine pad, a sanitary napkin, a panty liner, an incontinent garment, a diaper, etc. For purposes of explaining the invention herein, article 18 is depicted schematically as an elevated perspective view of a menstrual pad having certain portions cut-away for ease of viewing the components disposed inside the pad.

In accordance with the present invention, the article includes a liquid permeable topsheet (also known as a "cover"). The topsheet provides a smooth, non-irritating surface which contacts the skin of the user, who typically wears the garment. Desirably, the topsheet presents a supple an appealing texture to the touch. The topsheet should have sufficient coherent strength to maintain its integrity either wet or dry so that it can be cleanly and completely removed from the wearer's skin after use. Furthermore, the topsheet is sufficiently porous so as to permit ready passage of the bodily fluids through the topsheet to permit absorption of the bodily fluids by the article. The topsheet is desirably formed of fibers or filaments made from one or more of the materials taken from the group including: polyolefins, such as polypropylene, polyethylene and polybutylene; polyesters; polyamides; and cellusose. Using one or more of such materials, the topsheet can be formed as one of the following: woven webs, nonwoven webs, spunbond nonwoven webs, meltblown webs, spunbond-meltblown webs, thermally bonded carded webs, water laid webs, air laid webs, and conforms of one or more of the foregoing structures. Examples of coforms may be found in: (1) E.P. Application 89104799.5 filed March 17, 1989, and (2) E.P. Application 89104850.6 filed March 17, 1989, The contents of these applications, which have been assigned to the assignee of the present application, are hereby incorporated by reference.

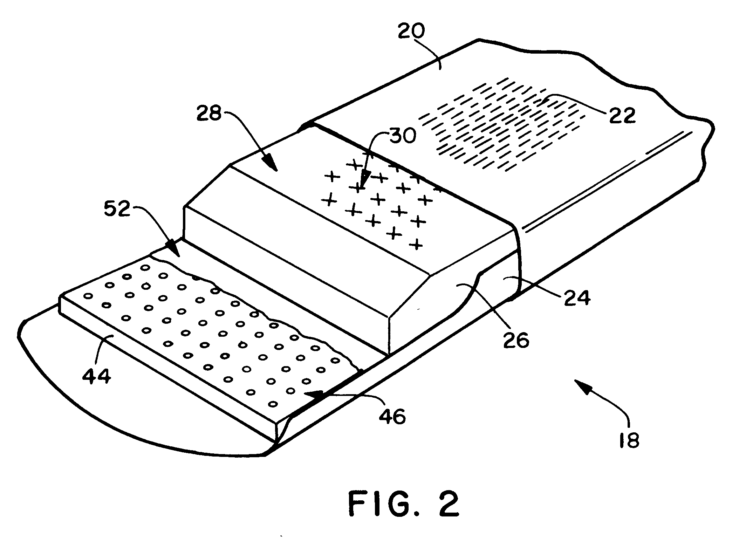

As embodied herein and shown for example in Figs. 1-7, article 18 includes a liquid permeable topsheet 20. As shown for example in Figs. 1-3 and 5-7, topsheet 20 can include a plurality of apertures 22 defined through a generally centrally located region of topsheet 20 (to avoid unduly complicating the Figs., only a few apertures are shown). Apertures 22 can take any of various forms, including circular holes as shown for example in Figs. 1, 5, and 6 and linear slits as shown for example in Figs. 2, 3, and 7. In use, topsheet 20 is positioned next to the body source of the bodily fluid.

In further accordance with the article of the present invention, a liquid impermeable backsheet 24 (also known as a "baffle") is provided. The backsheet 24 is desirably in the form of a sheet or film of material which is impervious to both liquid and vapor so as to eliminate any possibility of liquid or unpleasant odor escaping through the backsheet 24. Plastic material such as polypropylene, polyethylene or water proof paper are suitable materials for the backsheet. In an alternative embodiment, a waterproof film or layer may be laminated to the same material as the topsheet to form the backsheet material 24. In another alternative embodiment, the backsheet 24 may be a vapor permeable, but body fluid impermeable, layer of a blown microfiber web such as a spunbonded web of polypropylene. Such a material allows the passage of water vapor, and this escape of water vapor from the article adds to the general comfort of the user by preventing undue moisture buildup in the article yet still preventing leakage of the bodily material to undergarments or the like. In yet another alternative embodiment, the backsheet 24 could also be a fiber reinforced layer of a rubbery film-forming polymer such as butadiene-styrene.

Backsheet 24 and topsheet 20 are sufficiently joined to one another's respective peripheral edges to become integral components and effectively form an "envelope" which defines a cavity for receiving the other components of article 18. Any suitable adhering means may be employed such as glue or other like adhesives, heat seals, sonic welds, pressure sensitive adhesives, spot bonds, or crimps. Additionally, as is well known, backsheet 24 may further contain an adhesive strip or other positioning means (not shown), thereby allowing article 18 to be securely affixed to an undergarment or the like.

In yet further accordance with the article of the present invention, a liquid absorbent material 26 is disposed between the topsheet 20 and the backsheet 24, and has an upper surface 28 opposing, though not necessarily touching, the topsheet 20. The liquid absorbent material 26 is positioned so as to absorb the bodily fluid that passes through the topsheet 20. A number of suitable materials are known in the art for use as liquid absorbent material 26, and any of these known materials are within the scope of this invention. Desirably, the liquid absorbent material 26 includes any cellulose material. Examples of suitable liquid absorbent materials include cotton gauze, wood pulp, wood fluff, a coform (a poly/pulp blend), tissue wadding, superabsorbents, and combinations of any of the foregoing materials. As embodied herein and shown for example in Figs. 1-7, garment 18 includes one or more layers of liquid absorbent material 26 disposed between topsheet 20 and backsheet 24 and having an upper surface 28, which opposes topsheet 20.

It is generally known to those skilled in the art that bacteria present in menstrual fluid eventually metabolize the constituents of the fluid, such as proteins and fatty acids, and form byproducts which can be malodorous. When microorganisms present in menstrual fluid are allowed to grow in a used product such as a sanitary napkin, the number and amount of malodorous volatiles increases over the long term (at least about four hours).

In still further accordance with the present invention, odor protection over the long term (at least about 4 hours) is facilitated by providing at least one bacterial growth inhibitor 30. The bacterial growth inhibitor 30 is only effective if it directly contacts the bacteria in the menstrual fluid. Moreover, the longer the period of contact, the more effective is the antimicrobial action. Thus, in the present invention it is important to dispose the bacterial growth inhibitor 30 where it can directly contact :he bacteria in the menstrual fluid prior to the fluid being absorbed in liquid absorbent material 26. Accordingly, in the present invention, the bacterial growth inhibitor 30 generally is disposed between the topsheet 20 and the uppermost layer of liquid absorbent material 26. So located, liquid that passes through the topsheet 20 generally contacts the bacterial growth inhibitor 30 prior to contacting the liquid absorbent material. The bacteria in the menstrual liquid, for example, are inhibited from growing or are killed upon contacting the inhibitor.

Moreover, another important consideration of the present invention is that any antimicrobial agent provided in the article of the present invention, must be able to inhibit the growth of the bacteria present in menstrual fluid and on the article, while not disturbing the normal flora present on the skin's surface. The interposition of the topsheet 20 between the body and the bacterial inhibitor 30 greatly lessens undesirable disturbance of the flora present on the skin's surface.

As shown schematically in Figs. 1-7 for example, article 18 includes at least one bacterial growth inhibitor 30 (the presence of the bacterial growth inhibitor is schematically denoted by X's 30 in the Figs.) generally disposed between topsheet 20 and upper surface 28 of liquid absorbent material 26. This locates the bacterial growth inhibitor on the layer of liquid absorbent material 26 disposed closest to topsheet 20.

In selecting bacterial growth inhibitors suitable for use in the present invention, a number of factors should be kept in mind. Among the factors determining the mechanism required for the inhibition of growth of a particular bacteria are: (1) the species of bacteria; (2) the presence of special structures such as spores or capsules; and (3) the presence of extraneous materials such as blood and protein. Three ways in which antimicrobial agents act are: (1) to damage the cell membrane of the bacteria; (2) to irreversibly inactivate its proteins; and (3) to damage the nucleic acid. Antimicrobial agents that damage the cell membrane of the bacteria include surface-active disinfectants and phenolic compounds. Literature examples of surface active disinfectants are cationic agents such as quaternary ammonium compounds; anionic agents such as soaps or fatty acids; nonionic agents such as TRITON X-100™ (available from Rohm & Haas); and amphoteric agents such as glycine substituted with long chain alkyl amine group. Phenolic compounds cause membrane damage to the cell that results in leakage and lysis. Some examples of phenolic compounds having antimicrobial effects include: (1) cresols; (2) diphenyl compounds such as hexachlorophene; and (3) alcohols. Agents that denature proteins include acids and alkalies. Agents that modify functional groups of proteins and nucleic acids include heavy metals and oxidizing agents. The heavy metals that modify functional groups of proteins and nucleic acids include mercurials and silver. Oxidizing agents that modify functional groups of proteins and nucleic acids include halogens and hydrogen peroxide. The halogen oxidizing agents that exhibit antimicrobial effects include iodine and chlorine.

The bacterial growth inhibitor can include one or more inhibitors selected from the group that includes: chlorhexidine, quaternary ammonium compounds, cupric salts, chelating agents, parabens (methyl, ethyl, and propyl), chitin, and pH buffered materials. Disodium ethylenediaminetetraacetic acid (EDTA), mentioned in U.S. Patent No. 4,356,190, is a strong chelating agent and exhibits antimicrobial effects. Desirably, the bacterial growth inhibitor includes chlorhexidine digluconate, which is believed to be a suitable commercially available antimicrobial agent. Chlorhexidine digluconate inhibits the growth of a broad spectrum of bacteria. Chlorhexidine digluconate is also readily soluble in water. Thus, it is a relatively simple procedure to prepare a solution with chlorhexidine digluconate and apply the solution to various materials. Its effectiveness is not significantly reduced by the presence of organic matter such as blood. It clings to human skin, but is not absorbed through the skin. Finally, chlorhexidine digluconate binds well to many fabrics, particularly cotton and pulp.

One means of enhancing the inhibitor's efficacy is to impregnate the bacterial growth inhibitor into an insert, and then dispose the impregnated insert directly beneath the topsheet. As schematically shown in Figs. 1, 3, and 5-7 for example, bacterial growth inhibitor 30 is impregnated into an insert 32, which desirably is formed of a meltblown polypropylene web, or other suitable material such as cellulose pulp, and located immediately below topsheet 20. Moreover, insert 32 is desirably located beneath the region of topsheet 20 in which apertures 22 are defined.

An example of a meltblown polypropylene insert impregnated with a bacterial growth inhibitor can be made by preparing a solution of the antimicrobial containing hexanol as a wetting agent for a fabric formed as a polypropylene meltblown web. Then the polypropylene fabric is dipped in the solution, passed through a ringer, and dried in air. A meltblown insert has a good affinity for TEA, PYR, and propyl sulfide. In formulating a cellulose pulp insert, an appropriate amount of the bacterial growth inhibitor (chlorhexidine digluconate solution for example) is added to a dilute slurry of pulp, which is filtered and washed with water. The pulp is then dried and fluffed to yield a fluff pulp insert containing desirably 1.5% by weight of the antimicrobial agent. A uniform distribution of the antimicrobial agents can be obtained by each of the foregoing methods.

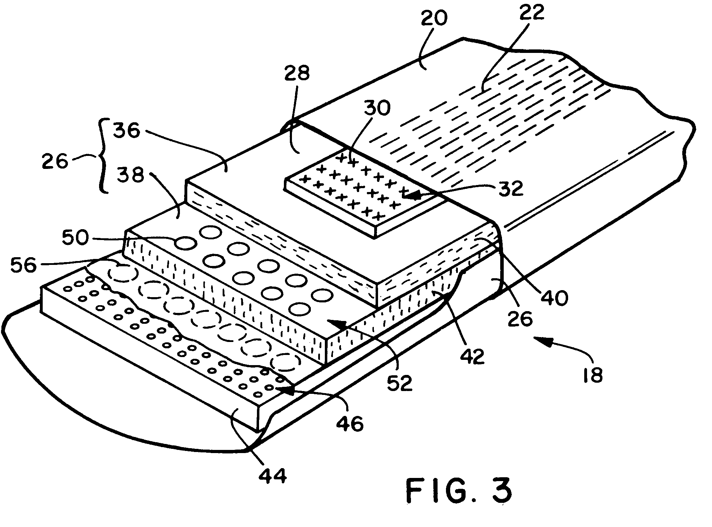

In an alternative embodiment such as shown schematically in Fig. 2 for example, bacterial growth inhibitor 30 is disposed upon upper surface 28 of liquid absorbent material 26, which is in turn directly disposed beneath topsheet 20. This can be accomplished by spraying the inhibitor onto upper surface 28 of liquid absorbent material 26. As schematically illustrated in Fig. 3 for example, 5 milligrams of chlorhexidine digluconate 30 can be sprayed in solution form onto a meltblown insert 32, which can be disposed between the uppermost surface 28 of liquid absorbent material 26 and topsheet 20, while disposed beneath apertures 22 of topsheet 20. The parabens and some of the other bacterial growth inhibitors are desirably handled in an ethanol/water mixture and sprayed onto an insert material forming a carrier system.

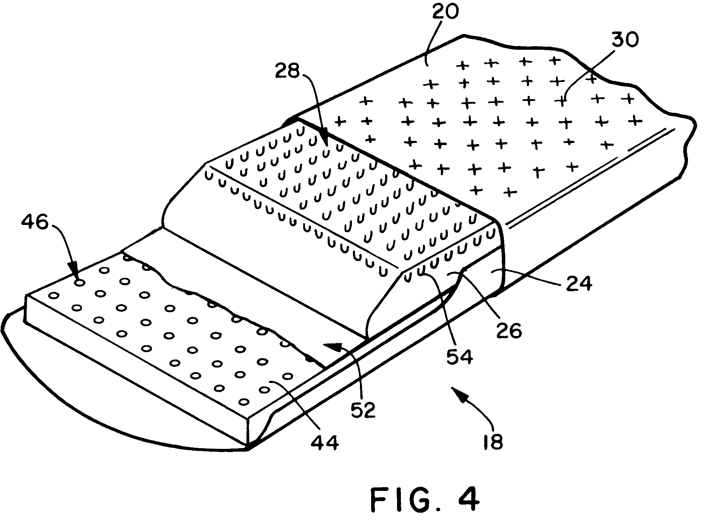

In a further alternative embodiment schematically illustrated in Fig. 4 for example, article 18 may also include a bacterial growth inhibitor 30 in the form of a surface segregated siloxane derivative incorporated into topsheet 20.

Odor control agents have been chosen for their ability to absorb a broad spectrum of odors present in menstrual fluid and its byproducts which is measured by an odor threshold. The odor threshold value is the minimum concentration in air of a volatile that is detectable by the human nose. Thus, to completely remove odor, the concentration of all the odorous volatiles must be brought below each individual odor threshold value. Using gas chromatography (GC), at least 50 different volatiles can be observed in used menstrual pads, and about 35 of the volatiles have a distinctive odor, many of the odors being recognized as either acidic or sulfurous. Of these 35, about 15 have a malodor. The malodorous volatiles from used menstrual pads are a complex mixture. Acidic, basic, and neutral volatile components are all present. Some of these volatiles have odor thresholds as low as one part per trillion. Used menstrual pads have been found to contain many malodorous chemical compounds, and these include triethylamine, pyridine, furaldehyde, isovaleric acids, and dimethyl sulfides.

Five volatile liquids were chosen to investigate the odor absorbing properties of materials for use in this invention, because each one: (1) represents a chemical class of compound found in used menstrual pads; (2) can easily be separated during gas chromatography; and (3) has a similar volatility to each of the other four chosen volatiles. Triethylamine (TEA) is an organic ammoniacal base. Pyridine (PYR) is an aromatic heterocyclic weak base. Furaldehyde (FUR) is an aromatic aldehyde. Isovaleric acid (IVA) is an aliphatic acid. Di-n-propyl sulfide (NPS) is an organic sulfide. A number of individual materials and combinations of materials were considered as odor control agents for the present invention. The individual materials considered as odor control agents for the present invention and the combinations of individual materials considered as odor control agents for :he present invention were evaluated in laboratory tests for their ability to control odor from the above-identified five volatiles (TEA, PYR, FUR, IVA, NPS).

One of the tests used in developing the present invention was the headspace analysis test, which indicates the efficiency of odor absorption after a fixed period of time. Briefly, the headspace analysis test places a known amount of the test substance in a vial and adds a known amount of test volatiles for a standard length time. A gas chromatograph is used to analyze the amount of each volatile that is present in a headspace above the test substance. The resulting number is compared to the controls.

Other tests used to develop the present invention include the odor breakthrough test and the capacity test. The odor breakthrough test measures the time it takes for an odor to breakthrough a given amount of test material at a known flow rate. The human nose is the detector. The odor breakthrough test is good for initial screening of materials to determine whether further investigation is warranted. The capacity test measures the total capacity (in percentages by weight of a material) of a material to absorb an odor. Thus the capacity test indicates the potential of a material to absorb an odor.

In the headspace analysis test used to develop the present invention, the odor removal ability of a potential odor control agent was determined using gas chromatography headspace analysis, described as follows:

A volatile mixture , referred to herein as the test volatile mixture, containing 100 microliters (hereafter abbreviated $\mu$l) di-n-propyl sulfide, 100 $\mu$l furaldehyde, 100 $\mu$l triethylamine, 200 $\mu$l isovaleric acid, and 100 $\mu$l of pyridine was prepared. A 10 $\mu$l volume of this volatile mixture was added by a pipetman, to a 40 milliliter Environmental Protection Agency (EPA) standard sample vial and sealed with a mininert screw cap. This serves as a blank to ensure the presence of all components of the volatile mixture.

A known quantity (e.g., 250 milligrams) of the odor control agent to be tested was placed in a 40-ml EPA vial. After the addition of 10 $\mu$l of the appropriate 5-component volatile mixture described above, the vial was sealed with a mininert screw cap and incubated at 37° C for approximately 4 hours. (When testing material in a sheet form, as opposed to a liquid form, an untreated sheet was used as the control, and in such case was run under the same conditions). After the allotted incubation period, a 10 $\mu$l headspace sample from each vial was injected into a Hewlett Packard 5890™ gas chromatograph using the following parameters:

Initial Time = 1.00 minutes
Initial Temp. = 35°C
Ramp Rate = 10°/minute
Final Temp. = 75°C
Final Time = 0.15 minutes
Inj. B = 175°C
Det. B = 300°C

| Oven Max. | = 300°C |
| Equib. Time | = 0.15 minutes |
| Flow B (He) | = 15.0-16.0 milliliters per minute |
| Range (Sig. 1) | = 4 |
| Zero (Sig. 1) | = 2 |
| Attn. (Sig. 1) | = 2 |

Pad prototypes containing odor absorbent materials were placed into 1000 ml Wheaton Purge and Trap Vessels that were attached to a water pump. A circulating water bath was used to pump 37°C water through the jacket of each Wheaton vessel. This system allowed the pad to be maintained at body temperature throughout the experiment. It also helped to volatilize the 5-component test volatile mixture. When testing the pad prototypes, an untreated pad was used as the control and run under the same conditions. A 50 µl volume of the test volatile mixture was placed on the top surface of each pad. The Wheaton vessels were sealed with teflon backed septa and allowed to incubate at 37°C for four hours. After the allotted incubation period, a 10 µl headspace sample from each Wheaton vessel was injected into a Hewlett Packard 5890™ gas chromatograph using the following parameters:

| Initial Time | = 1.00 minutes |
| Initial Temp. | = 35°C |
| Ramp Rate | = 10°/minute |
| Final Temp. | = 250°C |
| Final Time | = 10.00 minutes |
| Inj. B | = 175°C |
| Det. B | = 300°C |
| Oven Max | = 300°C |
| Equib. Time | = 0.15 |
| Flow B He | = 15.0-16.0 milliliters per minute |
| Range (Sig. 1) | = 3 |
| Range (Sig. 1) | = 2 |
| Range (Sig. 1) | = 2 |

A Hewlett Packard 3390A™ integrator was used to record the area count at the retention time of each of the five volatile components, and the percent absorption was calculated according to the following formula in which: X = the total area under the peak recorded by the integrator for the blank; Y = the total area under the peak recorded by the integrator for the sample.

$$\text{The percent absorption} = \frac{X - Y}{X} (100).$$

The present invention takes advantage of several factors identified in the testing. First, the location of an odor control agent within a product can be important to the odor removing efficiency of that odor control agent. For example, the placement of two grams of 1.0 M carbonate buffered pulp was found to be most effective on the underside of a top fluff layer of cellulose material.

A second factor learned from the testing was that certain combinations of odor control agents tend to increase the individual effectiveness of the component agents of the combination. In other words, the effect of the whole is greater than the sum of the individual effects of the parts. In Table 1, Example I, one example of the increased effectiveness of a combination, the combination of carbonate buffered pulp in a top fluff layer and the aluminosilicate molecular sieve powder sold by Union Carbide Corporation under the trade name "ABSCENTS" increases the adsorption of IVA and NPS when compared to the individual effectiveness of these two components. Thus, certain molecular sieves combined with carbonate buffered pulp showed increased selectivity for the sulfide once the acidic component of the volatile mixture was adsorbed.

A third factor confirmed by the testing was that wetting most odor control agents of the adsorbent class, renders them less efficient at removing odors. Thus, various artifices were tried in order to protect the odor control agents from contact with moisture. For example, in Example II, hydrophobically coating the carbon in the last combination mentioned in the above example, results in an increase in adsorption of IVA and TEA, but reduces the effectiveness against FUR and NPS. Moreover, a moisture-proof sheet can be disposed to shield moisture-sensitive odor control agents. Furthermore, an adsorbent might be placed where it is relatively shielded from contact with moisture.

TABLE I

PAD PROTOTYPE EVALUATIONS

GC Headspace Analysis - % Reduction of Volatile

Example I

| Sample | Volatiles | | | | |
|---|---|---|---|---|---|
| | TEA | PYR | FUR | IVA | NPS |
| Abscents™ (117 mg) | -56 | 27 | 25 | -23 | 24 |
| Carbonate buffered top fluff (150 mg) | -88 | -21 | -17 | 46 | 3 |
| Abscents™ and Carbonate buffered top fluff (117 mg + 150 mg) | -50 | 23 | 22 | 75 | 39 |

Example II

| Sample | Volatiles | | | | |
|---|---|---|---|---|---|
| | TEA | PYR | FUR | IVA | NPS |
| Carbonate buffered top fluff (150 mg) | 43 | 40 | 32 | 72 | 12 |
| Carbonate buffered top fluff and Carbon laminate (150 mg + 73 mg) | 0 | 44 | 75 | 21 | 74 |
| Carbonate buffered top fluff and hydrophobic coated Carbon laminate (150 mg + 73 mg) | 50 | 53 | 65 | 81 | 35 |

- means a percent increase of the volatile was observed.

In accordance with the article of the present invention, an odor control complex is disposed between the topsheet and the backsheet. The odor control complex is selected from a variety of odor control agents so that the selected agents form the odor control complex. When used in combination with one or more selected bacterial growth inhibitors, they reduce the malodor arising from a test volatile mixture, applied to the article, to levels undetectable by the human olfactory sense over periods of time up to and exceeding at least four hours.

As embodied herein, the odor control complex can include at least one pH buffered material. This pH buffered material can be one or more of the materials selected from the group including: acidic buffered pulp material, basic buffered pulp material, and neutral pH buffered pulp material. As embodied herein and shown schematically in Fig. 1 for example, article 18 includes at least one odor control agent (indicated schematically in Fig. 1 by the short horizontal lines 34). The odor control agent can be a pH buffered material. Desirably, the pH buffered material is integrated with liquid absorbent material 26.

Desirably, the buffer can be introduced into the slurry precursor used in the process of forming pulp inserts for the liquid absorbent material of the article. An example of an acidic buffer would include a citrate buffer. An example of a citrate buffered pulp is a combination of citric acid and its trisodium salt at a resulting pH of 5.5. This acidic pulp is effective at removing bases such as pyridine, and especially ammonia type compounds like triethylamine. To prepare a citrate buffer, 96.06 grams of citric acid can be dissolved in 500 milliliters of distilled water, and 294.10 grams sodium citrate can be dissolved into one liter of distilled water. Then, 150 milliliters of the citric acid solution can be added to the one 0.05.

An example of a basic buffer can include a carbonate buffer. An example of a basic pulp material can include a basic buffer, which is a combination of sodium carbonate and sodium bicarbonate at a pH of about 10.0 and prepared similarly to the way citrate buffered pulp is prepared. For example, a one molar solution of a carbonate buffer can be made by dissolving 21.2 grams of $Na_2CO_3$ in 200 milliliters of distilled water and 16.8 grams $NaHCO_3$ in 200 milliliters of distilled water. A ratio of 180 milliliters to 120 milliliters respectively, can be combined to obtain a pH of 10.0. This basic pulp buffer is an effective neutralizer of isovaleric acid, the acid component of the volatile mixture representative of menstrual fluid. The high pH of this basic pulp buffer also inhibits bacteria when in contact with same.

Alternatively, the buffer can be sprayed onto a formed pulp insert. During the testing for the present invention, the appropriate (acidic or basic) buffered solution was poured into a plant mister or TLC sprayer glassware, and each section of fluff to be tested was sprayed to a desired add-on weight, dried overnight on an open bench, and reassembled into a pad form. The best percent reduction of TEA was observed when the bottom layer of the liquid absorbent layer, such as a JOA[1] made layer, was sprayed with two grams of the citrate buffer.

Although acids and bases can effectively remove their opposites, they just as effectively produce large increases in their likes. For example, when sodium bicarbonate alone is introduced into the test solution of menstrual liquid volatiles, it increases the headspace concentration of triethylamine by 400%. Thus, when malodors consist of both acid and basic volatile components, the addition of a single acid or base is undesirable because it enhances the like odors.

In the present invention, the odor control complex can include both an acidic buffer and a basic buffer. Desirably, the acidic buffer and the basic buffer are carried by different portions of the liquid absorbent material. As embodied herein and shown schematically in Figs. 3, 5 and 6 for example, the liquid absorbent material 26 can include a top fluff layer 36 and a bottom fluff layer 38, with top fluff layer 36 being disposed between topsheet 20 and bottom fluff layer 38. As a result of the testing, it was concluded that the relative placement of acidic pulp buffer material was more effective in the bottom fluff layer located beneath a top fluff layer of basic buffer material. Thus, it is desirable to apply the basic buffer (the presence of the basic buffer is indicated schematically in Fig. 3 by the short horizontal lines 40) to the top fluff layer, while the acidic buffer (indicated schematically in Fig. 3 by the short vertical lines 42) is applied to the bottom fluff layer. So disposed, the top fluff layer 36 carrying the basic buffered pulp material 40 comes into contact with the bodily fluid before the bottom fluff layer 38, which carries the acidic buffered pulp material 42. This arrangement constitutes another instance of the relative location of two odor control agents (basic buffer pulp material 40 relative to acidic buffer pulp material 42) within article 18 serving to enhance their ability to eliminate odor. Note also that as shown schematically in Figs. 3, 5 and 6, the top fluff layer can be disposed adjacent to the bottom fluff layer. Results of these tests are in Table II.

[1] Made on JOA machinery.

## TABLE II

### PAD PROTOTYPE EVALUATIONS

#### GC Headspace Analysis - % Reduction of Volatile

| Sample | Volatiles | | | | |
|---|---|---|---|---|---|
| | TEA | PYR | FUR | IVA | NPS |
| Citrate buffered bottom fluff (563 mg) | 49 | 43 | 70 | -298 | 51 |
| Carbonate buffered bottom fluff (150 mg) | 0 | 52 | 75 | -111 | 42 |
| Carbonate buffered top fluff (150 mg) | 3 | 57 | 80 | -4 | 46 |
| Carbonate buffered top fluff and Citrate buffered bottom fluff (150 mg + 563 mg) | 73 | -78 | -28 | 45 | 6 |

- means a percent increase of the volatile was observed.

As embodied herein, the odor control complex can include at least one of a certain class of odor control agents known as adsorbents or molecular sieves. These are available in powder form and suitable as components for sheet form odor control members. As used herein, an adsorbent or molecular sieve chemically holds the odor causing molecule on the molecular surface of the adsorbent or sieve material. Suitable adsorbents or molecular sieves include: natural zeolites, synthetic zeolites, activated carbons, activated clays, activated aluminas, silica gels and aluminosilicates.

Certain of the adsorbents or molecular sieves suitable for use in this invention exhibit selective odor adsorption properties while others exhibit a relatively broad range of adsorption. This fact can be used to an advantage when selecting combinations of molecular sieves or combinations of molecular sieves and other odor control agents. Among the selective adsorbent materials are zeolites, which are crystalline "framework" structures containing aluminum and silicon atoms. They are crystals of ordered structures having uniform pore sizes and have adsorption properties of a lock and key nature, which means that the volatile must fit into the pore in order to be adsorbed. Zeolites are available as natural and synthetic forms and their specific absorption properties are based on their structure. Natural zeolite minerals include analcime, chabizet, clinoptilolite, erionite, laumonitite, mordenite, and phillipsite. Commercially available clinotilolite minerals include: Zeobrite™ (available from Zeotech Corporation of Albuquerque, New Mexico), Clinolite 20™ (available from Zeotech), XY zeolite™ (available from Teague Minerals of Adrian, Oregon), and SC zeolite™ (available from Teague Minerals). Selective odor adsorption properties are exhibited by molecular sieve 5A™ (available from Adsorbents and Desiccants Corporation of America, Gardena, California [hereafter ADCOA); Z5™ (available from Zeochem of Louisville, Kentucky); and Valfor C500-11™ (available from PQ Corporation of Valley Forge, Pennsylvania).

Multiple adsorbent materials are more effective at removing the complete spectrum of test volatiles. However, certain adsorbents have non-uniform pore sizes and undefined structures, and so result in random adsorption. (To make these more selective in their adsorption, the pore size can be changed by the addition of ions such as $Ca^{++}$, $Na^+$, $K^+$.) A relatively broad range of adsorption is exhibited by activated carbon, activated alumina, and each of the following commercially available brand name odor control agents: ABSCENTS™; 13X™ Molecular Sieve (available from ADCOA); XY™ zeolite and SC™ zeolite (available from Teague Minerals of Adrian, Oregon); F-200™ and Selexsorb CD 1/8™ (available from Aluminum Co. of America, Pittsburgh, PA); DD-430™ Activated Alumina (available from ADCOA); Z-10™ (available from Zeochem of Louisville, Kentucky); Carusorb 200™ (available from Carus Chemical Company of LaSalle, Illinois); and AX-21™ activated carbon (available from Anderson Development Co. of Adrian, Michigan).

A proprietary molecular sieve provided by Union Carbide is marketed under the trademark ABSCENTS™ for personal care use and SMELLRITE™ for household items. ABSCENTS™ is a white synthetic zeolite (an aluminosilicate structure) that readily adsorbs acid alone, but when the acid is in a complex mixture like menstrual fluid, it becomes less effective at absorbing the acid component of the complex mixture. A good natural zeolite replacement for ABSCENTS™ is Zeobrite™. The best odor adsorbers in humid conditions were activated carbon and ABSCENTS™. However, ABSCENTS™ and other zeolites are preferred over active carbon for their relative ease of processing.

Certain molecular sieves are more compatible with certain other molecular sieves or other odor control agents for controlling the broad spectrum of odors envisioned by this invention. In Table III, examples of the combination of Selexsorb CD 1/8™ (available from Aluminum Co. of America) and 13X™ Molecular Sieve (available from ADCOA) and the combination of 13X™ Molecular Sieve and DD-430™ Activated Alumina (available from ADCOA) are compatible molecular sieves of the broad range type.

## TABLE III

### COMMERCIAL ADSORBENT COMBINATIONS

#### GC Headspace Analysis - % Reduction of Volatile

| Sample | Volatiles | | | | |
|---|---|---|---|---|---|
| 250 mg TOTAL | TEA | PYR | FUR | IVA | NPS |
| CD | 95 | >99 | 97 | 98 | >99 |
| 13X | 99 | >99 | >99 | 95 | 99 |
| CD + 13X | >99 | >99 | >99 | >99 | >99 |
| DD-430 | 75 | 99 | >99 | >99 | >99 |
| DD-430 + 13X | 99 | >99 | >99 | >99 | >99 |

> means the detection limit of the instrument has been approached.

Other odor control agents which can be provided in powder or granular form suitable as components for a sheet form odor control member include at least one material selected from the group including: chitin, polymeric resin materials, ion exchange resins, Polymeric resins of insoluble cross linked polymers in bead form, and peat moss.

Chitin is a natural carbohydrate polymer that occurs primarily as a structural constituent in the exoskeletons of marine invertebrates and arthropods and to a lesser extent in other plant and animal forms such as fungi and yeasts. Structurally, it may be regarded as a derivative of cellulose in which the C-2 hydroxyl groups have been replaced by acetamido residues. Various chitins are particularly efficient for selectively removing particular volatiles. Protan 037-3xx-016 chitin and Protan PTL-260™ chitin (available from Protan Laboratories Inc. of Commack, NY) selectively remove TEA, PYR, and FUR. Sigma Chitin™ (available from Sigma Chemical Co.) shows selectivity for FUR. Chitin extracted in the laboratory from the exoskeletons of shrimps by choosing only the body parts (not the legs or tails) and by using only IN sodium hydroxide solution showed superior malodor absorbing properties to either of the above mentioned commercially available chitin materials. Its adsorption for TEA, PYR, FUR, and IVA was significant enough to be useful as an odor control agent in some embodiments of the present invention. Crab shell chitin showed a significant selectivity for IVA. Lab bleached chitin showed selectivity for PYR and NPS.

Polymer resins are also suitable for use in this invention as odor control agents. Their operation is generally known in the art and basically is an ion exchange mechanism. Similarly, polymeric resins of insoluble cross linked polymers in bead form are also suitable odor control agents. Desirable resins of this type are collectively known under the trademark "Amberlites" and are produced by Rohm & Haas Corp. Amberlite XAD-4™ and Amberlite XAD-7™ are especially suited for use in this invention.

"Ion exchange" material is a relatively broad definition and encompasses many types of materials, including the molecular sieves discussed above. It is within the scope of the odor control agents of this invention to include materials commonly referred to in the art as "ion exchangers" which have not been specifically discussed herein.

In further accordance with the present invention, the odor control complex can include a sheet form odor control member. Some of the odor adsorbents are powders and require a carrier system in order to be incorporated into a menstrual pad in an acceptable manner. A sheet form odor control member has been found to be an effective way of introducing powderous odor control agent materials into articles such as menstrual pads. In essence, a sheet form odor control member is a composite member that includes an odor adsorbent material in powder form adhered to a carrier substrate. Examples of such sheet form odor control members may be found in E.P. Application 90107020.1-2112 filed April 12, 1990.

Carrier substrates suitable as components of a sheet form odor control member include cellulose webs of softwood Kraft (BP-13 and BP-6),spunbond (SB) webs, spunbond-meltblown (SM) webs, and spunbond-meltblown-spunbond (SMS) webs. Coform web structures (cellulose-polypropylene) and creped wading are disfavored as carrier substrates because of their high absorbency and weak web structure. Two carrier substrates are favored for producing sheet form odor adsorbent members according to the present invention. One of the favored carrier substrates is a 33.91 g/m2 (1 oz./yd2) spunbond/meltblown (SM) laminate. The other favored carrier substrate is a cellulose web of softwood Kraft. spunbond/meltblown (SM) laminates and cellulose webs have slight odor reducing ability. A nonwoven SM web reduces bases, neutrals, and sulfur odors. meltblown webs formed of either KPR elastomeric (described hereafter) or polystyrene exhibit excellent adsorption of NPS. A cellulose web reduces basic and neutral odors. The cellulose web of softwood Kraft sheet absorbs triethylamine.

Two methods of applying the powderous odor control agent material to a web of material forming a carrier substrate are the saturation method and the coating method. As between the two, saturation is recommended over coating because of ease of preparation and because, as explained below, the binder component of the saturation application method has some odor reducing ability. The binder attaches the powderous odor adsorbent to the carrier substrate. In the saturation method, the combination of the binder and the powderous odor control agent is referred to as the saturant. Too much binder in the saturant can diminish the effectiveness of the odor control agent to reduce the malodorous volatiles. Too little binder can allow too much of the powderous odor control agent to detach from the carrier substrate and migrate within the article to locations where it becomes less effective. A 20:1 by weight ratio of odor control agent to binder gives a good balance of odor adsorption and adhesion of the odor control agent to the web which forms the carrier substrate.

Polyvinyl alcohol (PVOH) is a desirable binder in preparing sheet form odor control members because PVOH has some ability to reduce basic odors and the formulation of the saturant with PVOH is simple to prepare. Vinol 205™ PVOH (available from Air Products & Chemicals, Inc. of Allentown, PA) can be used in embodiments of the present invention. While latex can be used to make an acceptable saturant for a sheet form odor control member, the high binding efficiency of polyvinyl alcohol, the moderate cost of PVOH, and the ease of formulation of PVOH make it a preferred binder over latex.

## ABSCENTS™ (PVOH) SATURANT

|  | % SOLIDS | DRY | WET |
|---|---|---|---|
| Water | -- | -- | 229 |
| Triton X-100 sol'n | 10 | 10 | 100 |
| Abscents™ | 100 | 200 | 200 |
| Vinol 205 sol'n | 10 | 10 | 100 |
| TOTAL | 35* | 220 | 629 |

*Assuming BP-6 or SM with a dry add-on of 6.1 lb./1300 ft.²

(1 lb/ft² = 4.89 kg/m²)

15

Based on odor absorbing performance and the cost of production, two excellent performing sheet form odor control members are 108 milligrams of ABSCENTS™ delivered on a cellulose web and 60 milligrams of activated carbon delivered on a SM web as illustrated in Table IV. 120 milligram ABSCENTS™ on BP-6 with PVOH showed odor reduction of the test volatiles except IVA. 165 milligrams ABSCENTS™ on SM with PVOH showed odor reduction of the test volatiles except IVA and NPS. 133 milligrams ABSCENTS™ on SM with latex binder showed odor reduction of the test volatiles except IVA. Chitin on SB with PVOH binder showed odor reduction of the test volatiles except PYR. Only the nonwoven substrates containing activated carbon (60 milligrams activated carbon on spunbond meltblown with PVOH) were able to reduce the odor of IVA.

## TABLE IV

### GC Headspace Analysis - % Reduction of Volatile

| Loading/10 in$^2$ | Volatiles | | | | |
|---|---|---|---|---|---|
| | TEA | PYR | FUR | IVA | NPS |
| 0.108 g Abscents™ on cellulose web latex saturant | >99 | 99 | 94 | -187 | 96 |
| 0.120 g Abscents™ on cellulose web with PVOH saturant | 71 | 99 | 99 | -49 | 95 |
| 0.165 g Abscents™ on SM with PVOH saturant | 75 | 88 | 99 | -290 | -8 |
| 0.133 g Abscents™ on SM with latex saturant | 83 | 96 | 98 | -52 | 99 |
| Chitin w/PVOH on SB | 99 | -8 | 30 | 43 | 78 |
| 0.0598 g Act. Carbon on SM saturant | 88 | 73 | 89 | 92 | 92 |

Saturants containing dispersed activated carbon (Anderson AX-21™) are more efficient at removing odors than saturants containing a zeolite (ABSCENTS™). However, the zeolite saturant system may be desired over the activated carbon saturant system. The preference would be based on the greater difficulty, and thus higher cost, of preparing an activated carbon saturant versus a zeolite saturant. The saturation of activated carbon to a nonwoven or cellulose base web requires additional processing steps beyond those required to form a saturant with the zeolite. The use of a dispersant and an extra grinding step is needed to alleviate clumping of the carbon, and these extra steps increase the cost. Thus, a saturant which contains zeolite as the odor control agent and uses polyvinyl alcohol as the binder is desired for the sheet form odor control member of the present invention.

ACTIVATED CARBON SATURANT

| | % SOLIDS | DRY | WET |
|---|---|---|---|
| Activated Carbon Disp.* | 20 | 100 | 500 |
| Water | -- | -- | 100 |
| Triton X-100 sol'n | 10 | 10 | 100 |
| Vinol 205 sol'n | <u>10</u> | <u>10</u> | <u>100</u> |
| TOTAL | 15 | 120 | 800 |

*Assuming BP-6 (2X) or SM with a dry add-on of 4.1 lb./1300 ft$^2$.

lb/ft$^2$ = 4.89 kg/m$^2$)

| | % SOLIDS | DRY | WET |
|---|---|---|---|
| $NH_4OH$ | 28 | 2 | 7 |
| Water | -- | -- | 297 |
| Marasperse CBOS-4 | 100 | 20 | 20 |
| Anderson AX-21 | <u>35</u> | <u>100</u> | <u>286</u> |
| TOTAL | 20 | 122 | 610 |

Activated Carbon Dispersion:  Milled for one (1) hour.

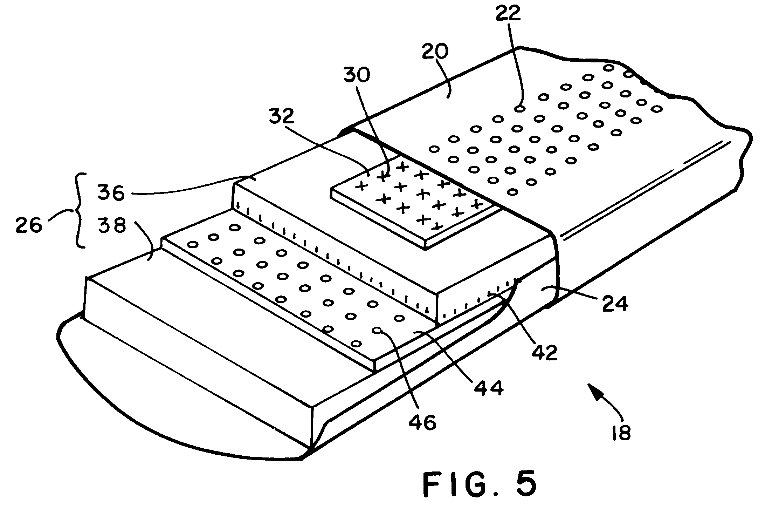

As embodied herein and schematically illustrated in Figs. 1 and 5 for example, a sheet form odor control member 44 including an odor adsorbent in powder form (schematically illustrated by the circles numbered 46) can be disposed between liquid absorbent material 26 and backsheet 24. Desirably, the powderous odor adsorbent material can include a synthetic zeolite material or activated carbon. The zeolite material can desirably be an aluminosilicate zeolite material such as ABSCENTS™. Moreover, as schematically illustrated in Fig. 1 for example, in addition to sheet form odor control member 44, the liquid absorbent material 26 can include an odor control agent (the presence of which being schematically illustrated by the horizontal lines numbered 34) such as a citrate buffer material, or chitin, or a carbonate buffer material .

Schematically in Fig. 5 for example, if the liquid absorbent material 26 includes a bottom fluff layer 38 and a top fluff layer 36 disposed between the topsheet 20 and the backsheet, the sheet form odor control member 44 is desirably disposed adjacent to the top fluff layer and between the top fluff layer and the bottom fluff layer. The top fluff layer can be disposed adjacent the bottom fluff layer, and the sheet form odor control member then is desirably disposed adjacent the top fluff layer.

As additionally shown schematically in Fig. 5 for example, the top fluff layer 36 can desirably carry on its underside a carbonate buffer (schematically illustrated by the vertical lines numbered 42) disposed on the top fluff layer's surface that is adjacent the sheet form odor control member 44. Desirably, the carbonate buffer can include sodium bicarbonate.

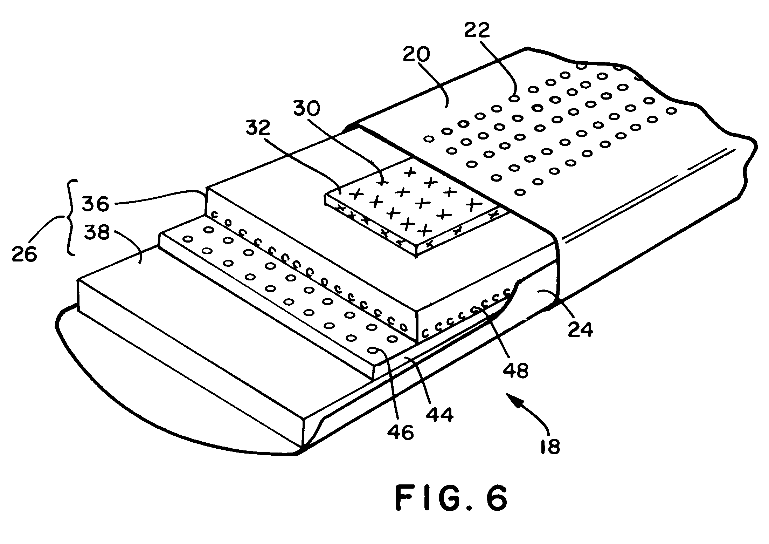

Activated carbon (AX-21), ABSCENTS™, and chitin were selected as three excellent representatives of broad based odor control agents based upon their ability to remove mixtures of volatile compounds. While chitin saturated on spunbond with polyvinyl alcohol showed odor reducing ability, the swelling of the chitin flakes during processing rendered it a less desirable odor adsorbent for incorporation into a sheet form odor control member. However, chitin can be provided in an embodiment of an article of the present invention in another manner. As shown schematically in Fig. 1 for example, odor control agent 34 can be provided as chitin in flake form, which can be mixed with the fluff pulp forming the liquid absorbent material 26. Alternatively, as shown schematically in Fig. 6 for example, chitin (schematically illustrated by the C's numbered 48) can be carried by the top fluff layer's surface that is adjacent the sheet form odor control member 44.

Most materials in the menstrual pad environment become moist during use by moisture from humidity or menstrual fluid. Moisture absorption reduces the ability of an odor adsorbent to remove malodorous volatiles.

In further accordance with the present invention, one or more artifices are employed for reducing the exposure of the odor control agents to moisture. Once moisture absorption is no longer a concern, the odor control agent can be placed within the article wherever needed and without regard for moisture.

As embodied herein, a hydrophobic coating applied to the odor control agents is one artifice for protecting the odor control agents from moisture deactivation. The hydrophobic coating must be permeable to the malodorous vapors which the odor control agent removes. A fluoropolymer can be used to provide a suitable hydrophobic coating. For example, an aqueous based solution of 1% by weight Zepel 6700™ fluoropolymer (available from DuPont) can be poured over adsorbent particles through a filtered gravity funnel. Care should be taken against using too much fluoropolymer, because it does prevent the volatiles from reaching the surface of the adsorbent and being removed thereby. The particulate odor control agents are placed on a watch glass and dried at 110° C for one hour. The cure temperature is the estimated glass transition temperature of the solution. Once this temperature is reached, the alignment of the hydrophobic charges are facing out, repelling water. Chitin, SMELLRITE™ and activated carbon can be coated with the fluorocarbon as described above. Coating of the commercial alternative natural zeolites CD, XY, and 13X can also be done, but is not as effective.

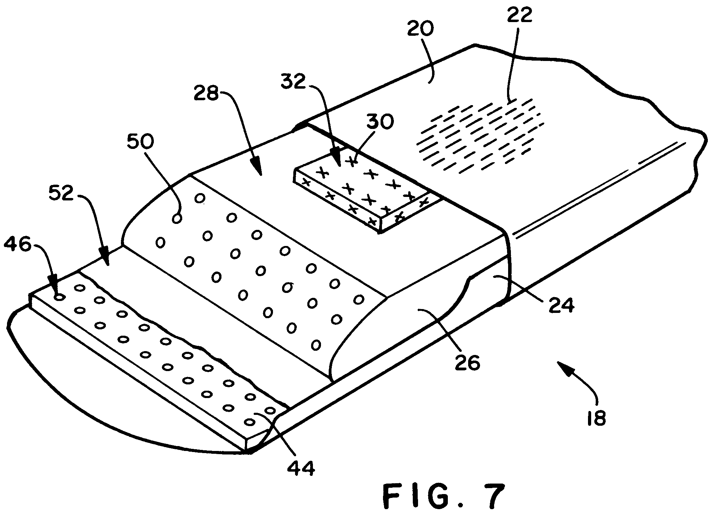

As shown schematically in Fig. 7 for example, article 18 can include hydrophobically coated adsorbent odor control agent (schematically illustrated by the circles numbered 50), such as a zeolite material, activated carbon, or chitin, disposed generally homogeneously throughout liquid absorbent material 26.

Additionally, the hydrophobic coating can be added directly onto a sheet form odor control member to coat the adsorbent material carried on the sheet. A hydrophobic coating can be applied to the zeolite material, the chitin, or the activated carbon material that comprises a sheet form odor control member. The hydrophobic coating (fluorocarbon) can be sprayed onto a sheet form odor control member using a bottle mister and cured as described above. As embodied herein and shown schematically in Fig. 5 for example, a hydrophobically coated sheet form odor control member 44 carrying activated carbon 46 can be placed into an article 18 between a top fluff layer 36 and a bottom fluff layer 38 such as a JOA[2] made layer. Moreover, a carbonate buffer 42 can be added to the top layer 36 of fluff. The coating and carbonate fluff are more effective than the untreated combination, as shown in Table V, except for FUR and NPS.

## TABLE V

### PAD PROTOTYPE EVALUATIONS

### GC Headspace Analysis – % Reduction of Volatile

| Sample | Volatile | | | | |
|---|---|---|---|---|---|
| | TEA | PYR | FUR | IVA | NPS |
| Carbonate buffered Top fluff (150 mg) | 43 | 40 | 32 | 72 | 12 |
| Carbonate buffered Top fluff and Carbon laminate (150 mg + 73 mg) | 0 | 44 | 75 | 21 | 74 |
| Carbonate buffered Top fluff and hydrophobic coated Carbon laminate (150 mg + 73 mg) | 50 | 53 | 65 | 81 | 35 |

– means a percent increase of the volatile was observed.

Another artifice or means of preventing wetting of the odor control agents is to incorporate an air permeable, but water repellent, web into the article at a location that shields the odor control agents from liquid. A material suitable to form a vapor permeable water repellent web is a meltblown web formed of elastomeric polymers.

[2] Made on JOA machinery.

A suitable elastomeric web can be formed of a polymer blend of 70% by weight Kraton G 1657 and 30% by weight polyethylene wax (hereinafter designated as Kraton Q 70/30 blend or KPR meltblown). Various "Kraton" blends for meltblowing purposes are described in U.S. Patent No. 4,657,802 to Morman, which is hereby incorporated herein by reference. When utilizing various of the "Kraton" materials (e.g., "Kraton" G) to form meltblown fibers or filaments, it is preferred to blend a polyolefin therewith, in order to improve meltblowing of such block copolymers. A polyolefin for blending with the "Kraton" G block copolymers is polyethylene, a desirable polyethylene being Petrothene Na601 obtained from U.S.I. Chemicals Company. Various elastomeric A-B-A' block copolymer materials are disclosed in U.S. Patent Nos. 4,323,534 to Des Marais and 4,355,425 to Jones, which are hereby incorporated herein by reference, and are available as "Kraton" polymers from the Shell Chemical Company. Meltblown webs of elastomeric polystyrene materials are excellent adsorbers of propyl sulfide (NPS) and triethylamine (TEA).

As embodied herein and shown schematically in Figs. 1, 2, 3, 4, and 7 for example, a web 52, which is vapor permeable but liquid impermeable, is disposed as a shield against wetting certain odor control agents. As shown schematically in Figs. 1, 2, 3, 4, and 7 for example, web 52 shields sheet form odor control member 44.

Yet another means or artifice for reducing the exposure of odor control agents to moisture is to strategically dispose the odor control agents within the product at sites where the incidence of moisture is less likely. Accordingly, as shown schematically in the embodiments shown in Figs. 1, 2, 3, 4, and 7 for example, the odor control agent (shown schematically in Figs. 1, 2, 3, 4, and 7 by the circles numbered 46) are disposed beneath liquid absorbent material 26 to reduce the wetting thereof by menstrual fluid.

During the investigations with chitin as an odor absorbing material, chitin was found to coagulate samples of menstrual fluid and blood. Presumably, the chitin binds to the blood cells which have negative charges and to proteins which are present in blood and menstrual fluid. This property of chitin is useful in this invention for aiding absorption of menstrual fluid and preventing leakage of the fluid from the article. Thus, in embodiments of the present invention utilizing chitin that has not been hydrophobically coated, the chitin is used as an odor control agent until it becomes wetted, at which point it then acts as a coagulating agent. When used as a coagulating agent, the chitin can be disposed so as to shield from contact with moisture those odor control agents which are adversely affected by contact with moisture. When used as a coagulating agent, the chitin is preferably in powder or particulate form, rather than flake form. In embodiments of the present invention utilizing chitin that has been hydrophobically coated to prevent wetting thereof, the chitin is used primarily as an odor control agent.

As shown schematically in Fig. 4 for example, embodiments of this invention can include uncoated chitin (schematically shown by the U's numbered 54) disposed within the liquid absorbent material 26 generally towards the topsheet 20. This disposition of the chitin enables it to directly contact the bodily fluid. As shown schematically in Fig. 7 for example, article 18 can further include hydrophobically coated chitin 50 disposed generally homogeneously throughout liquid absorbent material 26.

A desirable embodiment of the present invention will now be described. As shown schematically in Fig. 3 for example, an article 18 includes a liquid permeable topsheet 20 formed of 27.12 g/m² (.8 oz/yd²) spunbond and defining a plurality of apertures (schematically indicated by the short straight lines numbered 22) through the topsheet. A liquid impermeable backsheet 24 also is provided. A liquid absorbent material 26 is disposed between the topsheet and the backsheet. The liquid absorbent material is positioned so as to absorb liquid that passes through the topsheet. The liquid absorbent material includes a top fluff layer 36 that has about four grams of citrate buffered pulp (schematically indicated by the short horizontal straight lines numbered 40), prepared as described above. The liquid absorbent material further includes a 4.5 gram bottom fluff layer 38 such as a JOA³ made layer, and the top fluff layer is disposed between the topsheet and this bottom fluff layer. A bacterial growth inhibitor (schematically indicated by the X's numbered 30) is generally disposed between the topsheet and the liquid absorbent material. In this way, liquid passed through the topsheet generally contacts the bacterial growth inhibitor prior to contacting the liquid absorbent material. The bacterial growth inhibitor includes a meltblown insert 32 sprayed with a solution of about 5 milligrams of chlorhexidine digluconate. An odor control complex is disposed between the topsheet and the backsheet and includes about 1,000 milligrams of chitin (schematically indicated by the circles numbered 50), which is coated with sufficient fluoropolymer to render the coated chitin hydrophobic yet permeable to malodorous vapor. The coated chitin is disposed on the surface of the bottom fluff 38 layer facing toward the top fluff layer 36. The odor control complex also includes a sheet form odor control member 44, prepared as described above, that has about 117 milligrams of aluminosilicate zeolite material, ABSCENTS™, (schematically indicated by the circles numbered 46) bound to a cellulose web of softwood Kraft paper by a polyvinyl alcohol binder. The sheet form member is disposed between

³ Made on JOA machinery.

the bottom fluff layer 38 and the backsheet 24. The odor control complex also includes an air permeable water repellent web 52, such as a sheet of KPR meltblown, disposed between the bottom fluff layer 38 and the sheet form member 44. Finally, the odor control complex includes about 100 milligrams of a polymeric adsorbent material (schematically indicated by the dashed circles numbered 56). The polymeric adsorbent material is disposed between the sheet of KPR meltblown and the backsheet 24. A suitable polymeric adsorbent material for this purpose is AMBERLITE XAD-4™ available from Rohm & Haas. The surface tackiness of web 52 helps to hold particles 56 between web 52 and the carrier substrate of sheet form odor control member 44. Table VI discloses the results.

## TABLE VI

### GC Headspace Analysis – % Reduction of Volatile

| Sample | | Volatiles | | | |
|---|---|---|---|---|---|
| | TEA | PYR | FUR | IVA | NPS |
| Lab Sample 6/88 | >99 | 75 | 85 | 55 | 97 |

> means the detection limit of the instrument has been approached.

A second desirable embodiment of the present invention now will be described. As shown schematically in Fig. 5 for example, an article 18 includes a liquid permeable topsheet 20 formed of 0.8 ounces per square yard spunbond and defining a plurality of apertures (schematically indicated by the circles numbered 22) through the topsheet. A liquid impermeable backsheet 24 also is provided. A liquid absorbent material 26 is disposed between the topsheet and the backsheet. The liquid absorbent material is positioned so as to absorb liquid that passes through the topsheet. The liquid absorbent material includes a top fluff layer 36. The liquid absorbent material further includes a 4.5 gram bottom fluff layer 38 such as a JOA[4] made layer, and the top fluff layer is disposed between the topsheet and this bottom fluff layer. A bacterial growth inhibitor (schematically indicated by the X's numbered 30) is generally disposed between the topsheet and the liquid absorbent material. In this way, liquid passed through the topsheet generally contacts the bacterial growth inhibitor prior to contacting the liquid absorbent material. The bacterial growth inhibitor includes a meltblown insert 32 sprayed with a solution of about 5 milligrams of chlorhexidine digluconate. An odor control complex is disposed between the topsheet and the backsheet and includes about 150 milligrams to about 300 milligrams of carbonate buffered pulp (schematically indicated by the short vertical straight lines numbered 42) disposed in the top fluff layer of the liquid absorbent material. The odor control complex also includes a sheet form odor control member 44, prepared as described above, having about 234 milligrams of an odor control agent (schematically indicated by the circles numbered 46) such as aluminosilicate zeolite material, wherein the sheet form member is disposed between the top fluff layer 36 and the bottom fluff layer 38, as shown in Table VII.

[4] Made on JOA machinery.

## TABLE VII

### PAD PROTOTYPE EVALUATIONS

#### GC Headspace Analysis - % Reduction of Volatile

| Sample | Volatiles | | | | |
|---|---|---|---|---|---|
| | TEA | PYR | FUR | IVA | NPS |
| Abscents™ (234 mg) | -56 | 27 | 25 | -23 | 24 |
| Carbonate buffered Top fluff 150 mg) | -88 | -21 | -17 | 46 | 3 |
| Abscents™ and Carbonate buffered Top fluff (234 mg + 150 mg) | -50 | 23 | 22 | 75 | 39 |

-means a percent increase of the volatile was observed

A third desirable embodiment of the present invention now will be described. As shown schematically in Fig. 6 for example, an article 18 includes a liquid permeable topsheet 20 formed of 27.12 $g/m^2$ (.8 $oz/yd^2$) spunbond and defining a plurality of apertures (schematically indicated by the circles numbered 22) through the topsheet. A liquid impermeable backsheet 24 also is provided. A liquid absorbent material 26 is disposed between the topsheet and the backsheet. The liquid absorbent material is positioned thereby to absorb liquid that passes through the topsheet. The liquid absorbent material includes a top fluff layer 36 and a bottom fluff layer 38, and the top fluff layer is disposed between the topsheet and this bottom fluff layer. A bacterial growth inhibitor (schematically indicated by the X's numbered 30) is generally disposed between the topsheet 20 and the liquid absorbent material 26, and generally beneath apertures 22. In this way, liquid passed through the topsheet generally contacts the bacterial growth inhibitor prior to contacting the liquid absorbent material. The bacterial growth inhibitor includes a meltblown insert 32 sprayed with a solution of about 5 milligrams of chlorhexidine digluconate. An odor control complex is disposed between the topsheet and the backsheet and includes about 1,000 milligrams of an odor control agent such as chitin (schematically indicated by the C's numbered 48), which is disposed in the top fluff layer 36 of the liquid absorbent material. The odor control complex also includes a sheet form odor control member 44 disposed between the top fluff layer 36 and the bottom fluff layer 38. The sheet form member includes about 234 milligrams of an odor control agent (schematically indicated by the circles numbered 46) such as an aluminosilicate zeolite material like ABSCENTS™. See Table VIII.

## TABLE VIII

### PAD PROTOTYPE EVALUATIONS

#### GC Headspace Analysis - % Reduction of Volatile

| Sample | Volatiles | | | | |
|---|---|---|---|---|---|
| | TEA | PYR | FUR | IVA | NPS |
| Abscents™ and Chitin (234 mg + 1000 mg) | 65 | 80 | 93 | 93 | 66 |

Accordingly, the various embodiments discussed above are intended to illustrate how the combination and relative positioning of the components enhances odor removal performance over both the short and long term for a broad spectrum of undesirable odors encountered in the various environments served by the products constructed in accordance with the present invention. While several embodiments in accordance with the present invention have been shown, it is understood that the invention is not limited to these embodiments, but is susceptible of numerous changes and modifications as are appreciated by one having ordinary skill in the art upon reading this application

## Claims

1. An article (18) for absorbing bodily fluids and controlling malodor created by the presence of the bodily fluid in the article, the article comprising:
   a. a liquid permeable topsheet (20);
   b. a liquid impermeable backsheet (24);
   c. a liquid absorbent material (26) disposed between said topsheet (20) and said backsheet (24), said liquid absorbent material (26) being positioned to absorb liquid passed through said topsheet (20);
   d. at least one bacterial growth inhibitor (30) generally disposed between said topsheet (20) and said liquid absorbent material (26), whereby liquid passed through said topsheet (20) generally contacts said bacterial growth inhibitor (30) prior to contacting said liquid absorbent material;
   e. an odor control complex disposed between said topsheet (20) and said backsheet (24), said odor control complex being selected so that in combination with said bacterial growth inhibitor (30), malodor arising from a test volatile mixture, applied to the article (18) becomes reduced to levels undetectable by the human olfactory sense over periods of time of at least about four hours.

2. An article as in claim 1, wherein:
   said liquid absorbent material (26) includes:
   i) a top fluff layer (36), and
   ii) a bottom fluff layer (38),
   iii) said top fluff layer (36) being disposed between said topsheet (20) and said bottom fluff layer (38).

3. An article as in claim 2 wherein said odor control complex includes:
   i) an acidic buffer (42) carried by said top fluff layer (36) or said bottom fluff layer (38), and
   ii) a basic buffer (40) carried by said bottom fluff layer (38) or said top fluff layer (36).

4. An article as in claim 2 or 3, wherein:
   said odor control complex includes:
   i) a citrate buffer carried by said top fluff layer (36), and
   ii) a carbonate buffer carried by said bottom fluff layer (38).

5. An article as in one of claims 2 to 4, wherein:
   said odor control complex includes:
   i) a sheet form odor control member (44) including synthetic zeolite material or activated carbon material,
   ii) said sheet form odor control member (44) being disposed adjacent said top fluff layer (36) and between said top fluff layer (36) and said bottom fluff layer (38).

6. An article as in one of claims 2 to 5, wherein:
   said odor control complex includes:
   a carbonate buffer (42) disposed on the surface of said top fluff layer (36) adjacent said bottom fluff layer (38) or said sheet form odor control member (44).

7. An article as in claim 5, wherein:
   said odor control complex includes:
   chitin (48), said chitin being carried by said top fluff layer (36) adjacent said sheet form odor control member (44).

8. An article as in one of claims 5 to 7, further comprising:
   f. a coating covering said zeolite material, said coating being hydrophobic and permeable to malodorous vapor.

9. An article as in claim 7, further comprising:
   f. a coating covering said chitin, said coating being hydrophobic and permeable to malodorous vapor.

**10.** An article as in one of claims 5 to 9, further comprising:
f. a coating covering said activated carbon material, said coating being hydrophobic and permeable to malodorous vapor.

**11.** An article as in one of the preceding claims, wherein:
said odor control complex includes:
i) a sheet form member (44) including aluminosilicate zeolite material,
ii) said sheet form member (44) being disposed between said liquid absorbent material (26) and said backsheet (24), and
iii) at least one material of the group consisting of sodium bicarbonate, citrate buffer material, chitin and carbonate buffer material carried by said liquid absorbent material.

**12.** An article as in one of the preceding claims, wherein:
said odor control complex includes:
i) a sheet form member (44) including activated carbon material,
ii) said sheet form member being disposed between said liquid absorbent material (26) and said backsheet (24),
iii) a coating covering said activated carbon material, said coating being hydrophobic and permeable to malodorous vapor, and
iv) carbonate buffer material carried by said liquid absorbent material.

**13.** An article (18) for absorbing bodily fluids and controlling malodor created by the presence of the bodily fluid in the article, the article comprising:
a. a liquid permeable topsheet (20);
b. a liquid impermeable backsheet (24);
c. a liquid absorbent material (26) disposed between said topsheet (20) and said backsheet (24), said liquid absorbent material being positioned to absorb liquid passed through said topsheet (20), said liquid absorbent material including:
i) a top fluff layer (36),
ii) a bottom fluff layer (38), and
iii) said top fluff layer (36) being disposed between said topsheet (20) and said bottom fluff layer (38);
d. a bacterial growth inhibitor (30) generally disposed between said topsheet (20) and said liquid absorbent material (26), whereby liquid passed through said topsheet (20) generally contacts said bacterial growth inhibitor (30) prior to contacting said liquid absorbent material, said bacterial growth inhibitor including a meltblown insert (32) sprayed with about 5 milligrams of chlorhexidine digluconate in solution; and
e. an odor control complex disposed between said topsheet (20) and said backsheet (24), said odor control complex including:
i) carbonate buffered pulp in an amount ranging from about 150 milligrams to about 300 milligrams, said carbonate buffered pulp being disposed in said top fluff layer,
ii) a sheet form member (44) including about 234 milligrams of aluminosilicate zeolite material, and
iii) said sheet form member (44) being disposed between said top fluff layer (36) and said bottom fluff layer (38).

**14.** An article (18) for absorbing bodily fluids and controlling malodor created by the presence of the bodily fluid in the article, the article comprising:
a. a liquid permeable topsheet (20);
b. a liquid impermeable backsheet (24);
c. a liquid absorbent material (26) disposed between said topsheet and said backsheet, said liquid absorbent material being positioned to absorb liquid passed through said topsheet (20), said liquid absorbent material including:
i) a top fluff layer (36),
ii) a bottom fluff layer (38), and
iii) said top fluff layer (36) being disposed between said topsheet (20) and said bottom fluff layer (38);
d. a bacterial growth inhibitor (30) generally disposed between said topsheet (20) and said liquid absorbent material (26), whereby liquid passed through said topsheet (20) generally contacts said bacterial growth inhibitor (30) prior to contacting said liquid absorbent material (26), said bacterial growth inhibitor including a meltblown insert (32) sprayed with about 5 milligrams of chlorhexidine digluconate in solution; and

e. an odor control complex disposed between said topsheet (20) and said backsheet (24), said odor control complex including:

   i) about 1000 milligrams chitin being disposed in said top fluff layer,

   ii) a sheet form member (44) including about 234 milligrams of aluminosilicate zeolite material, and

   iii) said sheet form member being disposed between said top fluff layer (36) and said bottom fluff layer (38).

15. An article (18) for absorbing bodily fluids and controlling malodor created by the presence of the bodily fluid in the article, the article comprising:

   a. a liquid permeable topsheet (20);

   b. a liquid impermeable backsheet (34) joined to said topsheet and forming a cavity between said topsheet (20) and said backsheet (24);

   c. a liquid absorbent material (26) disposed in said cavity between said topsheet and said backsheet, said liquid absorbent material being positioned to absorb liquid passed through said topsheet (20);

   d. a bacterial growth inhibitor (30) generally disposed between said topsheet (20) and said liquid absorbent material (26), whereby liquid passed through said topsheet (20) generally contacts said bacterial growth inhibitor (30) prior to contacting said liquid absorbent material;

   e. an odor control complex disposed between said topsheet and said backsheet, said odor control complex including:

   i) chitin (48) disposed in said liquid absorbent material,

   ii) a sheet form odor control member (44) disposed between said liquid absorbent material (26) and said backsheet (24),

   iii) a liquid impermeable sheet (52) disposed between said liquid absorbent material (26) and said sheet form odor control member (44), and

   iv) a polymeric adsorbent material (56) disposed between said sheet (52) of liquid impermeable material and said backsheet (24).

16. An article as in claim 15, wherein:

   said bacterial growth inhibitor including a meltblown insert (32) sprayed with about 5 milligrams of chlorhexidine digluconate in solution.

17. An article as in one of claims 15 and 16, wherein:

   a. said topsheet (20) being formed of spunbond and defining a plurality of apertures (22) therethrough;

   b. said liquid absorbent material (36) including:

   i) a top fluff layer (36), said top fluff layer including about four grams of citrate buffered pulp, and

   ii) a bottom fluff layer (38),

   iii) said top fluff layer (36) being disposed between said topsheet (20) and said bottom fluff layer (38);

   c. said chitin having a mass of about 1000 milligrams;

   d. said chitin being coated with sufficient fluoropolymer to render said coated chitin hydrophobic yet permeable to malodorous vapor;

   e. said coated chitin being disposed on the surface of said bottom fluff layer (38) facing towards said top fluff layer (36);

   f. said sheet form odor control member (44) including:

   i) about 117 milligrams of aluminosilicate zeolite material,

   ii) said zeolite material being bound to a cellulose web of softwood Kraft paper,

   iii) said zeolite material being bound to said web by a polyvinyl alcohol binder;

   g. said sheet form odor control member (44) being disposed between said bottom fluff layer (38) and said backsheet (24);

   h. said sheet of liquid impermeable material (52) including a sheet of KPR meltblown;

   i. said sheet of KPR meltblown being disposed between said bottom fluff layer (38) and said sheet form odor control member (44);

   j. said polymeric adsorbent material having a mass of about 100 milligrams; and

   k. said polymeric adsorbent material (56) being disposed between said sheet (52) of KPR meltblown and said backsheet (24).

18. An article (18) for absorbing bodily fluids and controlling malodor created by the presence of the bodily fluid in the article, the article comprising:

a. a liquid permeable topsheet (20) formed of spunbond and defining a plurality of apertures (22) therethrough;

b. a liquid impermeable backsheet (24);

c. a liquid absorbent material (26) disposed between said topsheet and said backsheet, said liquid absorbent material being positioned to absorb liquid passed through said topsheet, said liquid absorbent material including:

i) a top fluff layer (36),

ii) a bottom fluff layer (38),

iii) said top fluff layer (36) being disposed between said topsheet (20) and said bottom fluff layer (38);

d. a bacterial growth inhibitor (30) generally disposed between said topsheet (20) and said liquid absorbent material (26), whereby liquid passed through said topsheet (20) generally contacts said bacterial growth inhibitor prior to contacting said liquid absorbent material (26), said bacterial growth inhibitor (30) including a meltblown insert sprayed with about 5 milligrams of chlorhexidine digluconate in solution;

e. an odor control complex disposed between said topsheet (20) and said backsheet (24), said odor control complex including:

i) about four grams of citrate buffered pulp disposed in said top fluff layer,

ii) about 1000 milligrams of chitin, said chitin being coated with sufficient fluoropolymer to render said coated chitin hydrophobic yet permeable to malodorous vapor,

iii) said coated chitin being disposed on the surface of said bottom fluff layer (38) facing towards said top fluff layer (36),

iv) a sheet form member (44) including about 117 milligrams of aluminosilicate zeolite material bound to a cellulose web of softwood Kraft paper by a polyvinyl alcohol binder,

v) said sheet form member (44) being disposed between said bottom fluff layer (38) and said backsheet (24),

vi) a sheet (52) of KPR meltblown disposed between said bottom fluff layer (38) and said sheet form member (44), and

vii) about 100 milligrams of polymeric adsorbent material (56), said polymeric adsorbent material being disposed between said sheet (52) of KPR meltblown and said backsheet (24).

19. An article (18) according to one of the preceding claims wherein the article is an incontinent garment.

20. An article (18) according to one of claims 1-18 wherein the article is a diaper.

FIG. 1

EP 0 510 619 A1

FIG. 2

EP 0 510 619 A1

FIG. 3

EP 0 510 619 A1

FIG. 4

EP 0 510 619 A1

FIG. 5

FIG. 6

FIG. 7

EP 0 510 619 A1

**European Patent Office**

# EUROPEAN SEARCH REPORT

Application Number

EP 92 10 6892

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| X<br>A | EP-A-0 389 023 (PROCTER & GAMBLE)<br>* page 3, line 46 - line 58 *<br>* page 4, line 21 - line 24 *<br>* page 9, line 45 - line 47 *<br>* page 10, line 22 - line 23 *<br>* page 11, line 21 - line 25; figure 2 *<br>--- | 1,19-20<br>2,5 | A61F13/15 |
| A | EP-A-0 376 761 (DIFINTER)<br><br>* abstract *<br>* page 7, line 25 - line 27; figure 1 *<br>--- | 1-2,<br>19-20 | |
| A | US-A-4 877 816 (K.MURABAYASHI AND K.MITANI)<br>* abstract *<br>* column 2, line 57 - column 3, line 1 *<br>* column 3, line 64 - line 68 *<br>--- | 1,19-20 | |
| A | WORLD PATENTS INDEX LATEST<br>Section PQ, Week 8828,<br>Derwent Publications Ltd., London, GB;<br>Class P32, AN 88-195904<br>& JP-A-63 135 501 (PIGEON AND ARAKAWA) 7 June 1988<br>* abstract * | 1,19-20 | TECHNICAL FIELDS SEARCHED (Int. Cl.5 )<br><br>A61L<br>A61F |

The present search report has been drawn up for all claims

| Place of search | Date of completion of the search | Examiner |
|---|---|---|
| THE HAGUE | 25 JUNE 1992 | NICE P. |

EPO FORM 1503 03.82 (P0401)